# EUROPEAN PATENT SPECIFICATION

(11) **EP 3 633 754 B1**
(45) Date of publication and mention of the grant of the patent: **27.01.2021**
(21) Application number: 18198757.9
(22) Date of filing: 05.10.2018
(51) Int. Cl.: H01M 2/02, H01M 10/48

(54) **BATTERY SYSTEM FOR A VEHICLE AND METHOD FOR DETECTING AN OVERHEAT SITUATION OF THE BATTERY SYSTEM**
BATTERIESYSTEM FÜR EIN FAHRZEUG UND VERFAHREN ZUR ERKENNUNG EINER ÜBERHITZUNGSSITUATION DES BATTERIESYSTEMS
SYSTÈME DE BATTERIE POUR UN VÉHICULE ET PROCÉDÉ DE DÉTECTION D'UNE SITUATION DE SURCHAUFFE DU SYSTÈME DE BATTERIE

(43) Date of publication of application: 08.04.2020
(73) Proprietor: Samsung SDI Co., Ltd., Gyeonggi-do (KR)
(72) Inventor: Erhart, Michael, 8054 Pirka-Seiersberg (AT)
(74) Representative: Gulde & Partner

(56) References cited:
- WO-A1-2016/086184
- DE-A1-102012 215 883

## Description

### Field of the Invention

The present invention relates to a battery system for a vehicle and a method for detecting an overheat situation of the battery system.

### Technological Background

In the recent years, vehicles for transportation of goods and peoples have been developed using electric power as a source for motion. Such an electric vehicle is an automobile that is propelled by an electric motor, using energy stored in rechargeable batteries. An electric vehicle may be solely powered by batteries or may be a form of hybrid vehicle powered by for example a gasoline generator. Furthermore, the vehicle may include a combination of electric motor and conventional combustion engine. In general, an electric-vehicle battery (EVB) or traction battery is a battery used to power the propulsion of battery electric vehicles (BEVs). Electric-vehicle batteries differ from starting, lighting, and ignition batteries because they are designed to give power over sustained periods of time. A rechargeable or secondary battery differs from a primary battery in that it can be repeatedly charged and discharged, while the latter provides only an irreversible conversion of chemical to electrical energy. Low-capacity rechargeable batteries are used as power supply for small electronic devices, such as cellular phones, notebook computers and camcorders, while high-capacity rechargeable batteries are used as the power supply for hybrid vehicles and the like.

In general, rechargeable batteries include an electrode assembly including a positive electrode, a negative electrode, and a separator interposed between the positive and negative electrodes, a case receiving the electrode assembly, and an electrode terminal electrically connected to the electrode assembly. An electrolyte solution is injected into the case in order to enable charging and discharging of the battery via an electrochemical reaction of the positive electrode, the negative electrode, and the electrolyte solution. The shape of the case, e.g. cylindrical or rectangular, depends on the battery's intended purpose. Lithium-ion (and similar lithium polymer) batteries, widely known via their use in laptops and consumer electronics, dominate the most recent group of electric vehicles in development. Rechargeable batteries may be used as a battery module formed of a plurality of unit battery cells coupled in series and/or in parallel so as to provide a high energy density, in particular for motor driving of a hybrid vehicle. That is, the battery module is formed by interconnecting the electrode terminals of the plurality of unit battery cells depending on a required amount of power and in order to realize a high-power rechargeable battery.

A battery pack is a set of any number of (preferably identical) battery modules. They may be configured in a series, parallel or a mixture of both to deliver the desired voltage, capacity, or power density. Components of battery packs include the individual battery modules, and the interconnections, which provide electrical conductivity between them.

For meeting the dynamic power demands of various electrical consumers connected to the battery system a static control of battery power output and charging is not sufficient. Thus, steady exchange of information between the battery system and the controllers of the electrical consumers is required. This information includes the battery systems actual state of charge (SoC), potential electrical performance, charging ability and internal resistance as well as actual or predicted power demands or surpluses of the consumers.

Battery systems usually comprise a battery management system (BMS) and/or battery management unit (BMU) for processing the aforementioned information. The BMS/BMU may communicate to the controllers of the various electrical consumers via a suitable communication bus, e.g. a SPI or CAN interface. The BMS/BMU may further communicate with each of the battery submodules, particularly with a cell supervision circuit (CSC) of each battery submodule. The CSC may be further connected to a cell connection and sensing unit (CCU) of a battery submodule that interconnects the battery cells of the battery submodule.

Thus, the BMS/BMU is provided for managing the battery pack, such as by protecting the battery from operating outside its safe operating area, monitoring its state, calculating secondary data, reporting that data, controlling its environment, authenticating it and/or balancing it.

To provide thermal control of the battery pack a thermal management system is required to safely use the at least one battery module by efficiently emitting, discharging and/or dissipating heat generated from its rechargeable batteries. If the heat emission/discharge/dissipation is not sufficiently performed, temperature deviations occur between respective battery cells, such that the at least one battery module cannot generate a desired amount of power. In addition, an increase of the internal temperature can lead to abnormal reactions occurring therein and thus charging and discharging performance of the rechargeable deteriorates and the life-span of the rechargeable battery is shortened. Thus, cell cooling for effectively emitting/discharging/dissipating heat from the cells is required.

Safety requirements, including the protection of passengers of a vehicle of harmful effluents of the battery system, such as toxic gases, smoke, or the like, are usually provided by a gas tight housing enclosing the battery modules and BMS/BMU for restricting the emission of such effluents.

However, while the system housing may protect the passengers from harmful fumes it complicates detection of critical states of the battery system that may lead to a catastrophic event, e.g. an explosion of the battery system. Such catastrophic events may be caused by an overheat situation. So far, the BMS/BMU could detect an overheat situation based only on acquired measurements regarding e.g. voltage, current and temperature. Hence, abnormal states which do not change the voltage, current, temperature or electrical insulation value have yet not been detectable. However, not all failures can be detected in a reasonable time. For example, badly screwed busbars may produce a high power dissipation and heat, which can overheat cells or other internal elements, which may end in destruction of cells or relays. Because of limitation of installation space and cost saving reasons not all cells and busbar interconnections could be supervised by temperature sensors.

WO 2016/086184 discloses a current interrupter triggered by a gas generating component, the generated gas delaminating a laminated connection for interrupting the electrical coupling between the electrode and its corresponding current collector. The current interrupter includes a layer containing a single gas generating component triggered by temperature.

It is thus an object of the present invention to overcome or reduce at least some of the drawbacks of the prior art and to provide a battery system that allows for an improved detection of an overheat situation of the battery system, particularly of such abnormal states that do not alter the voltage, current, or temperature of the total battery system, respectively temperature supervised cells.

### Summary of Invention

Embodiments of the present disclosure seek to solve at least one of the problems existing in the prior art to at least some extent. In particular, a battery system for a vehicle is provided comprising:
- one or more battery modules, each including a plurality of secondary battery cells;
- at least one gas sensor; and
- a housing for accommodating the one or more battery modules and the at least one gas sensor.

According to the present invention, at least parts of the exterior surface of the battery modules and/or at least parts of the interior surface of the housing are covered by a coating that is configured to emit at least one gaseous species when a temperature exceeds a predetermined temperature. The gas sensor is configured for detecting the at least one gaseous species.

According to the present invention, a thermally sensitive coating is deposited on surfaces of the battery system. When the temperature of the coating reaches a predetermined threshold, the coating starts emitting a gaseous species. Said gaseous species is then detected by a gas sensor provided in the interior space of the battery system. A battery management system BMS may be in direct communication with the gas sensor and, for example, may initiate safety and emergency measures, if necessary. In other words, a simple but robust kind of temperature control may be established within the housing encompassing the battery modules of the battery system. Thereby, overheating could be detected in areas of the battery system not being accessible for conventional temperature sensors.

The coating may include or consist of at least one of:
(i) a reactive system, which produces the at least one gaseous species by a chemical reaction at the predetermined temperature;
(ii) a compound having a boiling point or a sublimation point at the predetermined temperature, wherein the gaseous phase of the compound represents the gaseous species; and
(iii) a matrix system, which includes the gaseous species and is configured to emit the gaseous species at the predetermined temperature.

According to option (i), the coating includes or consists of one or more components which are converted to the gaseous species when the temperature reaches the predetermined temperature. For example, a compound may start to decompose to the gaseous species at a certain temperature or a chemical reaction between two compounds resulting information of the gaseous species will be thermally initiated when a certain temperature is reached.

According to option (ii), the coating includes a liquid or solid compound, which is transferred into its gaseous phase when the temperature reaches the boiling, respectively sublimation point of the compound.

According to option (iii), a matrix system will set the gaseous species free when the temperature reaches a certain temperature. For example, the gaseous species may be fixed in stable foam which decomposes when the temperature exceeds a certain temperature.

The gas sensor must be sensible for the gaseous species. In other words, gaseous species and gas sensor should be compatible.

The predetermined temperature is an inherent feature of the coating. That is, the skilled person may select the specific composition of the coating with respect to specific requirements of the battery system. For example, if the temperature at a certain surface of the battery module or housing should not exceed about 80 °C, the skilled person may consider deposition of a coating which will emit a detectable amount of the gaseous species when reaching a temperature of about 80 °C. He may thus, for example, select a compound for the coating having a boiling point or a sublimation point at around 80 °C, such like cyclohexane.

A suitable predetermined temperature may vary significantly for each battery system. Moreover, within a certain battery system different coatings sensible for different temperatures may be reasonable. In other words, the present invention may be useful with coatings having different predetermined temperatures covering a broad temperature range. However, a useful coating composition for a certain temperature may be simply identified by using a chemical database, like chemical abstracts, listening boiling points of compounds.

Preferably, the coating is provided on at least one of:
- an exterior surface of a battery cell;
- an electrical wiring of the battery system; and
- a cooling element for battery cells and battery modules.

For example, the coating may be deposited on parts the battery cell case or a cap plate covering an opening of the case. Thereby, supervision of overheating of each single battery cell may be easily established. In other words, battery cells, busbars, screws etc. of the battery module may be coated. In the case of an overheat situation of these parts the coating will degas characteristic compounds which will be detected by a gas sensor particularly configured for detecting this characteristic compounds.

The electrical wiring of the battery system includes the interconnection of battery cell terminals within a battery module, the interconnection of the battery modules, and the system terminals of the battery system. In particular, the electric wiring may include high or low current connectors and the coating is disposed on these current connectors. Thus, even overheating at a single current connector may be detected easily.

The coating may also be provided at a cooling element, like cooling plates. Hence, a failure of a cooling element may be detected prior to any harmful raise of temperature in the battery cells.

As mentioned above, the battery system may include different coatings at different places. For example, the coating of low current connectors can be different to the coating applied to high current connectors and they may emit different gas species at different temperatures. According to another embodiment, a single coating may also emit different gaseous species at different temperatures in order to distinguish different overheat situations according to the height of the overheat temperature.

According to a preferred embodiment, the battery system further includes means for forming a circulating air stream within the housing, wherein the air stream flows over the surfaces, where the coating is applied and the at least one gas sensor is placed within the air stream. Specifically, the means for forming the air stream may include a fan and the at least one gas sensor is placed within the air stream leaving the fan. In other words, by establishing a suitable air stream loop within the housing the gaseous species will be transferred to the gas sensor and it is not necessary to place the gas sensor next to the coating. Moreover, even a single gas sensor may be used for controlling the emission of several coatings since for security reasons it is mostly sufficient to know that a thermal fault occurred somewhere in the battery system.

According to another preferred embodiment, which may be also combined with any of the before mentioned embodiments, the battery system further comprises a battery management system BMS, connected to the at least one gas sensor, wherein the at least one gas sensor is configured for transmitting a control signal CS to the BMS in response to detecting the at least one gaseous species. Preferably, the battery system further comprises a battery disconnect unit BDU interconnected between at least one of the first and second system terminal and the at least one battery module, wherein the BMS is configured for transmitting a disconnect signal DS to the BDU in response to receiving the control signal CS, and wherein the BDU is configured for disconnecting at least one of a first and second system terminal of the at least one battery module in response to the disconnect signal DS. Thus, the detection of a certain gaseous species will immediately initiate safety measurements within the BMS itself.

According to another aspect of the invention there is provided a vehicle including the battery system as mentioned before.

According to a further aspect of the invention there is provided a method for detecting an overheat situation of a battery system comprising the steps of:
a) providing a battery system comprising:
   - a battery management system BMS;
   - one or more battery modules, each including a plurality of secondary battery cells;
   - at least one gas sensor; and
   - a housing for accommodating the one or more battery modules and the at least one gas sensor;
   wherein at least parts of the exterior surface of the battery modules and/or at least parts of the interior surface of the housing are covered by a coating that is configured to emit at least one gaseous species when a temperature exceeds a predetermined temperature and wherein the gas sensor is configured for detecting the at least one gaseous species;
b) detecting the at least one gas species with the gas sensor; and
c) transmitting at least one control signal CS from the gas sensor to the BMS in response to detecting the least one gas species.

Preferably, the method further comprises the steps:
d) determining the presence of an overheat situation in the battery system; and
e) controlling at least one countermeasure to the overheat situation.

In other words, the invention relates also to a method for detecting an overheat situation of a battery system in particular comprising at least one battery module interconnected between a first system terminal and a second system terminal by a plurality of high current connectors and comprising a housing with a plurality of exterior walls enclosing the at least one battery module and the plurality of high current connectors. The method comprises at least the steps of providing a coating that is configured to emit the at least one gaseous species at a temperature higher than a predetermined temperature threshold on least parts of the at least one battery module and/or the plurality of high current connectors; detecting a concentration of the least one gaseous species in the housing; and transmitting at least one control signal, CS, in response to detecting an excess concentration of the least one gaseous species.

The method of the present invention may be employed in battery systems employed for different purposes, and preferably in battery systems employed in a vehicle. Independent of the actual application of the battery system, the transmission of the control signal may severely intervene with the normal operation of the battery system. Hence, false positive events that erroneously indicate an abnormal condition of the battery system shall be prevented at any costs. On the other side first signs of imminent failures of components within the battery system should not be ignored and the battery system should be brought to a safe state as early as possible in order to reliably prevent catastrophic events, e.g. a battery fire. The method of the present invention provides such a reliable solution for detecting early signs of an overheat situation of a battery system as well as for estimating the state of health of safety critical components the battery system.

In the method of the present invention, the state of the battery system is determined in dependence of the concentration of the at least one gaseous species in the battery system. Particularly, a control signal is transmitted if the concentration of the at least one gaseous species exceeds a predetermined or learned threshold for that concentration. Preferably, a specific control signal out of a plurality of control signals is transmitted in response to detecting a predetermined or learned excess concentration for one or more gaseous species. Particularly preferred, a specific control signal out of a plurality of control signals is transmitted in response to detecting a predetermined combination of predetermined or learned excess concentrations for a plurality of gaseous species. The combination of excess concentrations that may occur in the battery system for an overheat situation may depend on coating of the specific parts having the overheat situation or of the specific temperature value of the overheat situation.

In the following Table 1 for a plurality of overheat situations characteristic gaseous species that may occur in such situation in an ordinary battery system are shown. Preferably, the at least one gaseous species emitted by the coating of the invention differs from these gaseous species in type and/or in magnitude of concentration in order to simplify the detection of an overheat situation and to avoid crosstalk.

**Table 1**

| | **hot spots, plastic smoldering** | **electric arc between live components** | **cell venting (thermal runaway)** | **fire** |
|---|---|---|---|---|
| **carbon dioxide (CO₂)** | **X** | | **X** | **X** |
| **carbon monoxide (CO)** | **X** | | **X** | **X** |
| **hydrogen (H₂)** | | | **X** | |
| **oxygen (O₂)** | | | | |
| **nitrogen oxides (NOₓ)** | | **X** | | |
| **ozone (O₃)** | | **X** | | |
| **water vapor (H₂O)** | **X** | | | |
| **Hydro-carbons** | **X** | | **X** | **X** |
| **smoke / particles** | **X** | **X** | **X** | **X** |

Particularly preferred, in response to the reception of the control signal(s) and/or the determination of the presence of an abnormal condition on the battery system, it is controlled to disconnect at least one of the first system terminal and the second terminal from the at least one battery module, to switch to an emergency mode of the battery system, to transmit a notification to a user of the battery system, to increase a cooling of the battery system, to reduce a state of power, SOP, of the battery system, and/or to trigger a fire extinguishing system for the battery system.

Therein the step of controlling or controlling to perform a countermeasure may comprise the step of transmitting a countermeasure control signal to an entity that shall actually perform the countermeasure. Preferably, the step of controlling or controlling to perform a countermeasure comprises the step of transmitting a countermeasure control signal via an interface of a battery management system to an exterior of the battery system. Particularly preferred, the step of controlling or controlling to perform a countermeasure comprises the step of transmitting, via an interface of the BMS, a disconnect signal as countermeasure control signal to a battery disconnect unit, BDU, for controlling the BDU to disconnect at least one of the first and second system terminal and the at least one battery module.

Further aspects of the present invention could be learned from the dependent claims or the following description.

### Brief Description of the Drawings

Features will become apparent to those of ordinary skill in the art by describing in detail an exemplary embodiment with reference to the attached drawing in which:
- Fig. 1: illustrates a schematic perspective view of a battery cell according to an embodiment of the present invention;
- Fig. 2: illustrates a schematic cross section of the battery cell shown in Fig.1;
- Fig. 3: shows a schematic illustration of a battery system according to an embodiment of the invention;
- Fig. 4: is an enlarged view on a part of the battery system illustrated in Fig. 3; and
- Fig. 5: is a flow chart illustrating a method for detecting an overheat situation of a battery system according to an embodiment of the present invention.

### Detailed Description of the Invention

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. As used herein, the term "and/or" includes any and all combinations of one or more of the associated listed items. Further, the use of "may" when describing embodiments of the present invention refers to "one or more embodiments of the present invention."

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

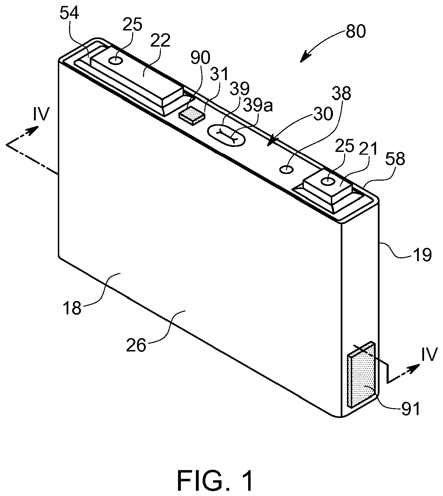

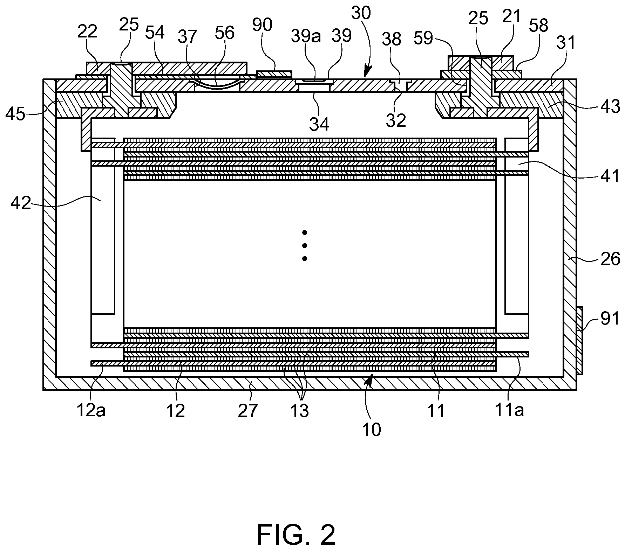

Fig. 1 is a perspective view illustrating a battery cell according to an exemplary embodiment and Fig. 2 is a cross-sectional view taken along the IV-IV line of Fig. 1.

As shown in Figs. 1 and 2, the battery cell 80 according to an embodiment may include an electrode assembly 10, and a case 26 for accommodating the electrode assembly 10, the case 26 containing an electrolyte. The battery cell 80 may also include a cap assembly 30 for sealing an opening of the case 26. The battery cell 80 will be described as a non-limiting example of a lithium ion secondary battery configured to have a prismatic shape.

The electrode assembly 10 may be formed as a jelly roll type electrode assembly by spirally winding a positive electrode 11 and a negative electrode 12 with a separator 13 therebetween. The positive electrode 11 and the negative electrode 12 may respectively include coated regions of current collectors formed of a thin metal foil, on which an active material may be coated, and may respectively include positive and negative electrode uncoated regions 11a and 12a of the current collectors on which no active material is coated. As a non-limiting example, the coated region of the positive electrode 11 may be formed by coating a base material formed of a metal foil, such as an aluminum foil, with an active material, such as transition metal oxide or the like. Also, the coated region of the negative electrode 12 may be formed by coating a base material formed of a metal foil, such as a copper or nickel foil, with an active material, such as carbon, graphite, or the like.

The positive electrode uncoated region 11a may be formed on one lateral end of the positive electrode 11 in a longitudinal direction of the positive electrode 11, and the negative electrode uncoated region 12a may be formed on one lateral end of the negative electrode 12 in a longitudinal direction of the negative electrode 12. The positive electrode uncoated region 11a and the negative electrode uncoated region 12a may be on sides that are opposite to each other with respect to the coated regions. Further, the separator 13 may include a plurality of separators, which may be spirally wound after the positive electrode 11, the negative electrode 12, and the separator 13 are alternately situated. The present invention is not limited thereto, and the electrode assembly 10 may be configured to have a structure including a plurality of sheets of the positive electrode 11, the separator 13, and the negative electrode 12 that are repeatedly stacked.

The electrode assembly 10 may be accommodated in the case 26 together with an electrolyte solution. The electrolyte solution may be made of a lithium salt, such as LiPF₆ or LiBF₄ with an organic solvent, such as EC, PC, DEC, EMC, or EMC. The electrolyte solution may be in a liquid, solid, or gel state. The case 26 may be configured to have a substantially cuboidal shape, and an opening may be formed at one side thereof. The case 26 may be formed of a metal, such as aluminum.

The case 26 may include a bottom surface 27 having a substantially rectangular shape, and may include a pair of first lateral walls, that are the wide side surfaces 18, 19, and a pair of second lateral walls, that are narrow side surfaces, connected vertically to end portions of the bottom surface 27, respectively, to form a space for accommodating the electrode assembly 10. The first lateral walls 18, 19 may face each other, and the second lateral walls may be positioned to face each other and may be connected to the first lateral walls 18, 19. A length of an edge at which the bottom surface 27 and a first lateral wall 18, 19 are connected to each other may be longer than that of an edge at which the bottom surface 27 and the second lateral wall are connected to each other. Preferably, adjacent first and second lateral walls enclose an angle of about 90°.

The cap assembly 30 may include a cap plate 31 for covering the opening of the case 26 by being bonded to the case 26, and may include a positive terminal 21 (first terminal) and a negative terminal 22 (second terminal), which are externally protruded from the cap plate 31 to be electrically connected to the positive electrode 11 and the negative electrode 12, respectively. The cap plate 31 may be configured to have a shape of a plate that may be extended in one direction, and that may be bonded to the opening of the case 26. The cap plate 31 may include an injection hole 32 and a vent hole 34 that communicate with an interior of the cap assembly 30. The injection hole 32 may be configured to allow the injection of the electrolyte solution, and a sealing cap 38 may be mounted thereon or therein. Further, a vent member 39 including a notch 39a, which may be opened due to a predetermined pressure may be mounted to or in the vent hole 34.

The positive terminal 21 and the negative terminal 22 may be mounted to protrude upward from the cap plate 31. The positive terminal 21 may be electrically connected to the positive electrode 11 via a current collecting tab 41, and the negative terminal 22 may be electrically connected to the negative electrode 12 via a current collecting tab 42. A terminal connecting member 25 for electrically connecting the positive terminal 21 and the current collecting tab 41 may be mounted between the positive terminal 21 and the current collecting tab 41. The terminal connecting member 25 may be inserted into a hole formed at the positive terminal 21, such that a lower portion thereof may be welded to the current collecting tab 41.

A gasket 59 for sealing may be mounted between the terminal connecting member 25 and the cap plate 31, while being inserted into the hole through which the terminal connecting member 25 may extend. Further, a lower insulating member 43, into which the lower portion of the terminal connecting member 25 may be inserted, may be mounted under the cap plate 31. A connecting plate 58 for electrically connecting the positive terminal 21 and the cap plate 31 may be mounted between the positive terminal 21 and the cap plate 31. The terminal connecting member 25 may be inserted into the connecting plate 58. Accordingly, the cap plate 31 and the case 26 may be positively electrified.

A terminal connecting member 25 for electrically connecting the negative terminal 22 and the current collecting tab 42, which is similar to the terminal connecting member 25, may be installed between the negative terminal 22 and the current collecting tab 42. The terminal connecting member 25 may be inserted into the hole formed at the negative terminal 22, such that an upper portion and a lower portion of the terminal connecting member 25 may be, respectively, welded to the negative terminal 22 and to the current collecting tab 42. A gasket for sealing, which is similar to the gasket 59, may be mounted between the negative terminal 22 and the cap plate 31, while being inserted into the hole through which the terminal connecting member 25 may extend. Further, a lower insulating member 45, which is for insulating the negative terminal 22 and the current collecting tab 42 from the cap plate 31, may be mounted under the cap plate 31.

An upper insulating member 54 for electrically insulating the negative terminal 22 and the cap plate 31 may be mounted between the negative terminal 22 and the cap plate 31. The terminal connecting member 25 may be inserted into a hole formed at the upper insulating member 54. The cap assembly 30 may include a short-circuiting hole 37, and a short-circuiting member 56 that may short-circuit the positive electrode 11 and the negative electrode 12 installed in the short-circuiting hole 37. The short-circuiting member 56 may be between the upper insulating member 54 and the cap plate 31, and the upper insulating member 54 may be configured to have a cutout that may be formed at a position corresponding to the short-circuiting member 56. The short-circuiting member 56 may overlap the negative terminal 22 exposed through the cutout, and may be separately located.

Further, the short-circuiting member 56 may be between the negative terminal 22 and the vent hole 34, and may be located closer to the negative terminal 22 than to the vent hole 34. The short-circuiting member 56 may include a convexly curved portion that curves toward the electrode assembly 10, and may include an edge portion may be formed at an outside of the curved portion and fixed to the cap plate 31. The short-circuiting member 56 may be deformed to cause a short-circuit when an internal pressure of the battery cell 80 rises. In other words, the internal pressure of the battery cell 80 may rise when a gas is generated by an unwanted reaction in the battery cell 80. When the internal pressure of the battery cell 80 is increased to be higher than a level (e.g., a predetermined level), the curved portion may be deformed to be concavely curved toward an opposite direction, thereby causing the short-circuiting member 56 to contact the negative terminal 22 to cause a short-circuit.

According to the exemplary embodiment illustrated in Figures 1 and 2, a first coating 90 is disposed on a part of the cap plate 31 and a second coating 91 is disposed on a part of a side surface of the case 26. However, the arrangement of the coatings 90, 91 is not limited thereto. Coatings may be deposited on any surface of the battery cell 80 which will be accessible for air within a housing of the battery system as will be explained in detail further below.

The coatings 90, 91 are configured to emit at least one gaseous species when a temperature exceeds a predetermined temperature. Here, the first and second coatings 90, 91 have the same composition, i.e. will emit the same gaseous species when the temperature exceeds the same predetermined temperature. However, the first and second coatings 90, 91 may also have different compositions emitting different gaseous species at for example different temperatures. Such coatings 90, 91 may include or consist of at least one of:
(i) a reactive system, which produces the at least one gaseous species by a chemical reaction at the predetermined temperature;
(ii) a compound having a boiling point or a sublimation point at the predetermined temperature, wherein the gaseous phase of the compound represents the gaseous species; and
(iii) a matrix system, which includes the gaseous species and is configured to emit the gaseous species at the predetermined temperature.

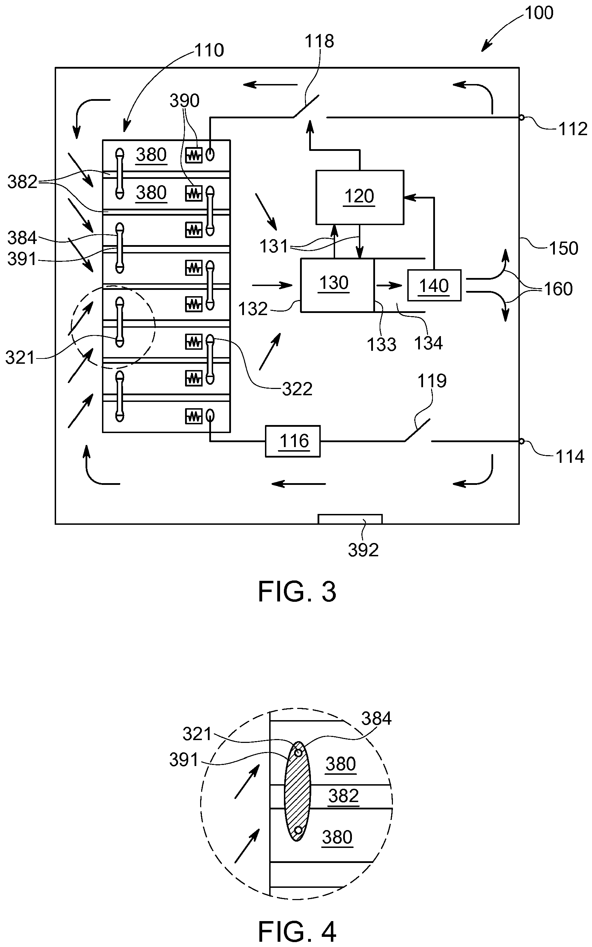

Fig. 3 illustrates in a highly schematically way a top view on an exemplary battery system 100 according to the invention. The battery system 100 includes a stack of battery cells 380 being assembled and electrically interconnected to form a battery module 110. The battery cells 380 of the battery module 110 may be similar to the battery cell 80 described above with reference to Figs. 1 and 2. Cooling plates 382 are disposed between adjacent battery cells 380. A coating 390 similar to the coating 90 of the embodiment of Fig. 1 is disposed on the top surface of each of the battery cells 380.

The negative and positive terminals 321, 322 of neighboring battery cells 380 are interconnected by high current connectors 384. As shown in detail in Fig. 4, a coating 391, which allows emission of at least one gaseous species when a temperature exceeds a predetermined temperature, is disposed on the entire upper surface of the current connectors 384. Thus, the battery module 110 is interconnected between a first system terminal 112 and a second system terminal 114 by a plurality of high current connectors 384. The interconnection may further include a current sensor 116 and relays 118, 119 for connecting, respectively disconnecting the system terminals 112, 114 with the battery module 110.

The battery system 100 further comprises a conventionally battery management system BMS 120. The BMS 120 may communicate to the controllers of the various electrical consumers via a suitable communication bus, e.g. a SPI or CAN interface. The BMS 120 may further communicate with a cell supervision circuit (CSC) of each battery module 110 (not shown). Thus, the BMS 120 is provided for managing the battery system 100, such as by protecting the battery cells 380 from operating outside their safe operating area, monitoring their state, calculating secondary data, reporting that data, etc. Here, the BMS communicates also with a battery disconnect unit BDU.

According to the illustrated embodiment of the present invention, the battery system 100 further includes a fan 130. Via data lines 131 the BMS 120 may regulate and supervise the activity and status of the fan 130. An inlet opening 132 of the fan 130 is provided on a side of the fan 130 facing the battery module 110. An outlet opening 133 is positioned opposite to the inlet opening 132 and opens out into a flow channel 134. A gas sensor 140 is positioned within the flow channel 134.

Furthermore, a housing 150 is provided for accommodating the battery modules 110, the BMS 120, the fan 130, and the gas sensor 140. The housing may include a rigid frame structure and cover elements for hermetically closing the battery system 100. The housing 150 will usually be mounted to the underbody of an electric vehicle (not shown). According to the exemplary embodiment, a coating 392, which allows emission of at least one gaseous species, when a temperature exceeds a predetermined temperature, is disposed on an interior surface of the housing 150.

When the fan 130 is switched on, a circulating air stream indicated by arrows 160 is formed within the housing 150. The air stream flows over each surface, where the coatings 390, 391, 392 are applied and the gas sensor 140 is placed within said air stream. In case the temperature at the coatings 390, 391, 392 will exceed a certain temperature, a gaseous species will be emitted and transferred by the air flow towards the gas sensor 140. The gas sensor 140 may be selective for the gaseous species.

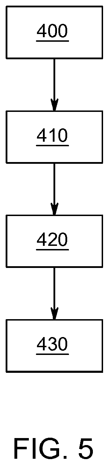

As schematically illustrated in the flow chart of Fig. 5, detection of the gaseous species by the gas sensor 140 causes output of a control signal CS (Step 400), followed by transmission of CS to the BMS 120 (Step 410). In response to the CS, the BMS 120 may initiate in Step 420 disconnection between the first and second system terminals 112, 114 by means of the battery disconnect unit BDU, i.e. the BMS 120 is configured for transmitting a disconnect signal DS to the BDU in response to receiving the control signal CS, and the BDU is configured for disconnecting at least one of a first and second system terminal 112, 114, for example by switching relay 118 (Step 430). Thus, the BMS 120 determines on basis of a control signal CS of the gas sensor 140 the presence of an overheat situation in the battery system 100 and controls at least one countermeasure to said overheat situation, for example disconnection of the battery module 110.

### Reference signs

- 10: electrode assembly
- 11: positive electrode
- 11a: positive electrode uncoated region
- 12: negative electrode
- 12a: negative electrode uncoated region
- 13: separator
- 18: first lateral side surface
- 19: second lateral side surface
- 21, 321: positive cell terminal
- 22, 322: negative cell terminal
- 25: terminal connecting member
- 26: case
- 27: bottom surface
- 30: cap assembly
- 31: cap plate
- 32: injection hole
- 34: vent hole
- 37: short-circuiting hole
- 38: sealing cap
- 39: vent member
- 39a: notch
- 41, 42: current collecting tab
- 43, 45: lower insulating member
- 54: upper insulating member
- 56: short-circuiting member
- 58: connecting plane
- 59: gasket
- 80, 380: battery cell
- 90, 91, 390, 391, 392: coating for emitting gaseous species
- 100: battery system
- 110: battery module
- 112, 114: first and second system terminals
- 116: current sensor
- 118, 119: relay
- 120: battery management system BMS
- 130: fan
- 132: inlet opening
- 133: outlet opening
- 134: flow channel
- 140: gas sensor
- 150: housing
- 160: arrows indication circulating air stream
- 382: cooling plate
- 384: high current connector

## Claims

1. A battery system (100) for a vehicle, comprising:
- one or more battery modules (110), each including a plurality of secondary battery cells (80, 380);
- at least one gas sensor (140); and
- a housing (150) for accommodating the one or more battery modules (110) and the at least one gas sensor (140);
wherein at least parts of the exterior surface of the battery modules (110) and/or at least parts of the interior surface of the housing (150) are covered by a coating (90, 91, 390, 391, 392) that is configured to emit at least one gaseous species when a temperature exceeds a predetermined temperature and wherein the gas sensor (140) is configured for detecting the at least one gaseous species.

2. The battery system of claim 1, wherein the coating (90, 91, 390, 391, 392) includes or consists of at least one of:
(i) a reactive system, which produces the at least one gaseous species by a chemical reaction at the predetermined temperature;
(ii) a compound having a boiling point or a sublimation point at the predetermined temperature, wherein the gaseous phase of the compound represents the gaseous species; and
(iii) a matrix system, which includes the gaseous species and is configured to emit the gaseous species at the predetermined temperature.

3. The battery system of claim 1, wherein the coating (90, 91, 390, 391, 392) is provided on at least one of:
- an exterior surface of a battery cell (80, 380);
- an electrical wiring of the battery system (100); and
- a cooling element for battery cells (80) and battery modules (110).

4. The battery system of claim 3, wherein the electrical wiring includes current connectors (384) and wherein the coating (391) is disposed on the current connectors (384).

5. The battery system of claim 1, further including means for forming a circulating air stream within the housing (150), wherein the air stream flows over the surfaces, where the coating (390, 391, 392) is applied and the at least one gas sensor (140) is placed within the air stream.

6. The battery system of claim 5, wherein the means for forming the air stream include a fan (130) and the at least one gas sensor (140) is placed within the air stream leaving the fan (130).

7. The battery system of any one of the preceding claims, further comprising a battery management system BMS (120), connected to the at least one gas sensor (140), wherein the at least one gas sensor (140) is configured for transmitting a control signal CS to the BMS (120) in response to detecting the at least one gaseous species.

8. The battery system of claim 7, further comprising a battery disconnect unit BDU interconnected between at least one of a first and second system terminal (112, 114) and the at least one battery module (110), wherein the BMS (120) is configured for transmitting a disconnect signal DS to the BDU in response to receiving the control signal CS, and wherein the BDU is configured for disconnecting at least one of a first and second system terminal (112, 114) of the at least one battery module (110) in response to the disconnect signal DS.

9. A vehicle including a battery system (100) according to any one of the preceding claims.

10. A method for detecting an overheat situation of a battery system (100) comprising the steps of:
a) providing a battery system comprising:
- one or more battery modules (110), each including a plurality of secondary battery cells (80, 380);
- at least one gas sensor (140);
- a housing (150) for accommodating the one or more battery modules (110) and the at least one gas sensor (140); and
- a battery management system BMS (120),
wherein at least parts of the exterior surface of the battery modules (110) and/or at least parts of the interior surface of the housing (150) are covered by a coating (90, 91, 390, 391, 392) that is configured to emit at least one gaseous species when a temperature exceeds a predetermined temperature and wherein the gas sensor (140) is configured for detecting the at least one gaseous species;
b) detecting the at least one gas species with the gas sensor (140); and
c) transmitting at least one control signal CS from the gas sensor (140) to the BMS (120) in response to detecting the least one gas species.

11. The method of claim 10, further comprising the steps:
d) determining the presence of an overheat situation in the battery system (100); and
e) controlling at least one countermeasure to the overheat situation.

## Patentansprüche

1. Ein Batteriesystem (100) für ein Fahrzeug, aufweisend:
- ein oder mehrere Batteriemodule (110), die jeweils eine Vielzahl von Sekundärbatteriezellen (80, 380) aufweisen;
- zumindest einen Gassensor (140); und
- ein Gehäuse (150) zum Aufnehmen des einen oder der mehreren Batteriemodule (110) und des zumindest einen Gassensors (140);
wobei zumindest Teile der Außenfläche der Batteriemodule (110) und/oder zumindest Teile der Innenfläche des Gehäuses (150) mit einer Beschichtung (90, 91, 390, 391, 392) bedeckt sind, die konfiguriert ist, um zumindest eine Gasart zu emittieren, wenn eine Temperatur eine vorbestimmte Temperatur überschreitet, und wobei der Gassensor (140) konfiguriert ist, um die zumindest eine Gasart zu erkennen.

2. Das Batteriesystem nach Anspruch 1, wobei die Beschichtung (90, 91, 390, 391, 392) zumindest eines von Folgendem aufweist oder daraus besteht:
(i) ein reaktives System, das die zumindest eine Gasart durch eine chemische Reaktion bei der vorbestimmten Temperatur erzeugt;
(ii) eine Verbindung, die einen Siedepunkt oder einen Sublimationspunkt bei der vorbestimmten Temperatur aufweist, wobei die Gasphase der Verbindung die Gasart darstellt; und
(iii) ein Matrixsystem, das die Gasart aufweist und konfiguriert ist, um die Gasart bei der vorbestimmten Temperatur zu emittieren.

3. Das Batteriesystem nach Anspruch 1, wobei die Beschichtung (90, 91, 390, 391, 392) auf zumindest einem von Folgendem bereitgestellt wird:
- einer Außenfläche einer Batteriezelle (80, 380);
- einer elektrischen Verdrahtung des Batteriesystems (100); und
- eines Kühlelements für Batteriezellen (80) und Batteriemodule (110).

4. Das Batteriesystem nach Anspruch 3, wobei die elektrische Verdrahtung Stromverbinder (384) aufweist und wobei die Beschichtung (391) auf den Stromverbindern (384) angeordnet ist.

5. Das Batteriesystem nach Anspruch 1, ferner aufweisend Mittel zum Ausbilden eines zirkulierenden Luftstroms im Gehäuse (150), wobei der Luftstrom über die Oberflächen strömt, wo die Beschichtung (390, 391, 392) aufgebracht ist, und der zumindest eine Gassensor (140) im Luftstrom positioniert ist.

6. Das Batteriesystem nach Anspruch 5, wobei die Mittel zum Ausbilden des Luftstroms einen Ventilator (130) aufweisen und der zumindest eine Gassensor (140) im Luftstrom, der aus dem Ventilator (130) ausströmt, positioniert ist.

7. Das Batteriesystem nach einem der vorhergehenden Ansprüche, ferner aufweisend ein Batteriemanagementsystem BMS (120), das mit dem zumindest einen Gassensor (140) verbunden ist, wobei der zumindest eine Gassensor (140) konfiguriert ist, um in Reaktion auf das Erkennen der zumindest einen Gasart ein Steuersignal CS zum BMS (120) zu übertragen.

8. Das Batteriesystem nach Anspruch 7, ferner aufweisend eine Batterietrenneinheit BDU, die zwischen zumindest einen eines ersten und zweiten Systemanschlusses (112, 114) und das zumindest eine Batteriemodul (110) geschaltet ist, wobei das BMS (120) konfiguriert ist, um in Reaktion auf das Empfangen des Steuersignals CS ein Trennsignal DS zur BDU zu übertragen, und wobei die BDU konfiguriert ist, um in Reaktion auf das Trennsignal DS zumindest einen eines ersten und zweiten Systemanschlusses (112, 114) des zumindest einen Batteriemoduls (110) zu trennen.

9. Ein Fahrzeug, aufweisend ein Batteriesystem (100) nach einem der vorhergehenden Ansprüche.

10. Ein Verfahren zur Erkennung einer Überhitzungssituation eines Batteriesystems (100), das die folgenden Schritte aufweist:
a) Bereitstellen eines Batteriesystems, aufweisend:
- ein oder mehrere Batteriemodule (110), die jeweils eine Vielzahl von Sekundärbatteriezellen (80, 380) aufweisen;
- zumindest einen Gassensor (140); und
- ein Gehäuse (150) zum Aufnehmen des einen oder der mehreren Batteriemodule (110) und des zumindest einen Gassensors (140); und
- ein Batteriemanagementsystem BMS (120),
wobei zumindest Teile der Außenfläche der Batteriemodule (110) und/oder zumindest Teile der Innenfläche des Gehäuses (150) mit einer Beschichtung (90, 91, 390, 391, 392) bedeckt sind, die konfiguriert ist, um zumindest eine Gasart zu emittieren, wenn eine Temperatur eine vorbestimmte Temperatur überschreitet, und wobei der Gassensor (140) konfiguriert ist, um die zumindest eine Gasart zu erkennen;
b) Erkennen der zumindest einen Gasart mit dem Gassensor (140); und
c) Übertragen zumindest eines Steuersignals CS vom Gassensor (140) zum BMS (120) in Reaktion auf das Erkennen der zumindest einen Gasart.

11. Das Verfahren nach Anspruch 10, ferner aufweisend die folgenden Schritte:
d) Bestimmen des Vorliegens einer Überhitzungssituation im Batteriesystem (100); und
e) Steuern zumindest einer Gegenmaßnahme gegen die Überhitzungssituation.

## Revendications

1. Système de batterie (100) pour un véhicule, comprenant :
- un ou plusieurs modules de batterie (110), contenant chacun une pluralité d'éléments de batterie secondaire (80, 380) ;
- au moins un capteur de gaz (140) ; et
- un boîtier (150) pour loger les un ou plusieurs modules de batterie (110) et l'au moins un capteur de gaz (140) ;
dans lequel au moins des parties de la surface extérieure des modules de batterie (110) et/ou au moins des parties de la surface intérieure du boîtier (150) sont recouvertes d'un revêtement (90, 91, 390, 391, 392) qui est configuré pour émettre au moins une espèce gazeuse quand la température dépasse une température prédéterminée, et dans lequel le capteur de gaz (140) est configuré pour détecter l'au moins une espèce gazeuse.

2. Système de batterie selon la revendication 1, dans lequel le revêtement (90, 91, 390, 391, 392) comprend ou est constitué d'au moins l'un parmi :
(i) un système réactif, qui produit l'au moins une espèce gazeuse par une réaction chimique à la température prédéterminée ;
(ii) un composé ayant un point d'ébullition ou un point de sublimation à la température prédéterminée, où la phase gazeuse du composé représente l'espèce gazeuse ; et
(iii) un système de matrice, qui contient l'espèce gazeuse et est configuré pour émettre l'espèce gazeuse à la température prédéterminée.

3. Système de batterie selon la revendication 1, dans lequel le revêtement (90, 91, 390, 391, 392) est disposé sur au moins l'un parmi :
- une surface extérieure d'un élément de batterie (80, 380) ;
- un câblage électrique du système de batterie (100) ; et
- un élément de refroidissement pour éléments de batterie (80) et modules de batterie (110).

4. Système de batterie selon la revendication 3, dans lequel le câblage électrique comprend des connecteurs de courant (384) et dans lequel le revêtement (391) est disposé sur les connecteurs de courant (384).

5. Système de batterie selon la revendication 1, comprenant en outre des moyens pour former un courant d'air en circulation à l'intérieur du boîtier (150), dans lequel le courant d'air s'écoule sur les surfaces, où le revêtement (390, 391, 392) est appliqué et l'au moins un capteur de gaz (140) est placé à l'intérieur du courant d'air.

6. Système de batterie selon la revendication 5, dans lequel les moyens pour former le courant d'air comprennent un ventilateur (130) et l'au moins un capteur de gaz (140) est placé à l'intérieur du courant d'air quittant le ventilateur (130).

7. Système de batterie selon l'une quelconque des revendications précédentes, comprenant en outre un système de gestion de batterie BMS (120), connecté à l'au moins un capteur de gaz (140), dans lequel l'au moins un capteur de gaz (140) est configuré pour transmettre un signal de commande CS au BMS (120) en réponse à la détection de l'au moins une espèce gazeuse.

8. Système de batterie selon la revendication 7, comprenant en outre une unité de déconnexion de batterie BDU interconnectée entre au moins l'une parmi des première et deuxième bornes de système (112, 114) et l'au moins un module de batterie (110), dans lequel le BMS (120) est configuré pour transmettre un signal de déconnexion DS à la BDU en réponse à la réception du signal de commande CS, et dans lequel la BDU est configurée pour déconnecter au moins l'une parmi des première et deuxième bornes de système (112, 114) de l'au moins un module de batterie (110) en réponse au signal de déconnexion DS.

9. Véhicule comprenant un système de batterie (100) selon l'une quelconque des revendications précédentes.

10. Procédé pour détecter une situation de surchauffe d'un système de batterie (100), comprenant les étapes consistant à :
a) disposer d'un système de batterie comprenant :
- un ou plusieurs modules de batterie (110), contenant chacun une pluralité d'éléments de batterie secondaire (80, 380) ;
- au moins un capteur de gaz (140) ; et
- un boîtier (150) pour loger les un ou plusieurs modules de batterie (110) et l'au moins un capteur de gaz (140) ; et
- un système de gestion de batterie BMS (120),
dans lequel au moins des parties de la surface extérieure des modules de batterie (110) et/ou au moins des parties de la surface intérieure du boîtier (150) sont recouvertes d'un revêtement (90, 91, 390, 391, 392) qui est configuré pour émettre au moins une espèce gazeuse quand la température dépasse une température prédéterminée, et dans lequel le capteur de gaz (140) est configuré pour détecter l'au moins une espèce gazeuse ;
b) détecter l'au moins une espèce gazeuse avec le capteur de gaz (140) ; et
c) transmettre au moins un signal de commande CS du capteur de gaz (140) au BMS (120) en réponse à la détection de l'au moins une espèce gazeuse.

11. Procédé selon la revendication 10, comprenant en outre les étapes consistant à :
d) déterminer la présence d'une situation de surchauffe dans le système de batterie (100) ; et
e) commander au moins une contre-mesure à la situation de surchauffe.
